# FASCICULE DE BREVET EUROPEEN

(11) **EP 1 423 708 B1**
(45) Date de publication et mention de la délivrance du brevet: **29.11.2006**
(21) Numéro de dépôt: 02781373.2
(22) Date de dépôt: 06.09.2002
(51) Int. Cl.: G01N 33/92, C07K 14/47

(54) **COMPOSITIONS ET METHODES POUR LE DOSAGE DE L'AA4RP**
ZUSAMMENSETZUNGEN UND VERFAHREN ZUM BESTIMMEN VON AA4RP
COMPOSITIONS AND METHODS FOR AA4RP ASSAY

(30) Priorité: 07.09.2001 FR 0111598; 12.08.2002 FR 0210205
(43) Date de publication de la demande: 02.06.2004
(73) Titulaire: Genfit, 59120 Loos (FR)
(72) Inventeur: NAJIB, Jamila, F-59211 Santes (FR); MAJD, Zouher, F-59700 Marcq en Baroeul (FR)
(74) Mandataire: Tezier Herman, Béatrice
(86) Numéro de dépôt international: PCT/FR2002/003041
(87) Numéro de publication internationale: WO 2003/023408

(56) Documents cités:
- WO-A-00/03013
- WO-A-00/37491
- WO-A-01/00803
- WO-A-01/53486
- WO-A-01/79446

### LISTE DE SEQUENCES

<110> GENFIT SA
<120> Compositions et Methodes pour le dosage de l'AA4RP
<130> B0157WO
<140>
   <141>
<160> 2
<170> PatentIn Ver. 2.1
<210> 1
   <211> 366
   <212> PRT
   <213> Homo sapiens
<400> 1
   Met Ala Ser Met Ala Ala Val Leu Thr Trp Ala Leu Ala Leu Leu Ser
   1               5                   10                  15
   Ala Phe Ser Ala Thr Gln Ala Arg Lys Gly Phe Trp Asp Tyr Phe Ser
               20                  25                  30
   Gln Thr Ser Gly Asp Lys Gly Arg Val Glu Gln Ile His Gln Gln Lys
           35                  40                  45
   Met Ala Arg Glu Pro Ala Thr Leu Lys Asp Ser Leu Glu Gln Asp Leu
       50                  55                  60
   Asn Asn Met Asn Lys Phe Leu Glu Lys Leu Arg Pro Leu Ser Gly Ser
   65                  70                  75                  80
   Glu Ala Pro Arg Leu Pro Gln Asp Pro Val Gly Met Arg Arg Gln Leu
                   85                  90                  95
   Gln Glu Glu Leu Glu Glu Val Lys Ala Arg Leu Gln Pro Tyr Met Ala
               100                 105                 110
   Glu Ala His Glu Leu Val Gly Trp Asn Leu Glu Gly Leu Arg Gln Gln
           115                 120                 125
   Leu Lys Pro Tyr Thr Met Asp Leu Met Glu Gln Val Ala Leu Arg Val
       130                 135                 140
   Gln Glu Leu Gln Glu Gln Leu Arg Val Val Gly Glu Asp Thr Lys Ala
   145                 150                 155                 160
   Gln Leu Leu Gly Gly Val Asp Glu Ala Trp Ala Leu Leu Gln Gly Leu
                   165                 170                 175
   Gln Ser Arg Val Val His His Thr Gly Arg Phe Lys Glu Leu Phe His
               180                 185                 190
   Pro Tyr Ala Glu Ser Leu Val Ser Gly Ile Gly Arg His Val Gln Glu
           195                 200                 205
   Leu His Arg Ser Val Ala Pro His Ala Pro Ala Ser Pro Ala Arg Leu
       210                 215                 220
   Ser Arg Cys Val Gln Val Leu Ser Arg Lys Leu Thr Leu Lys Ala Lys
   225                 230                 235                 240
   Ala Leu His Ala Arg Ile Gln Gln Asn Leu Asp Gln Leu Arg Glu Glu
                   245                 250                 255
   Leu Ser Arg Ala Phe Ala Gly Thr Gly Thr Glu Glu Gly Ala Gly Pro
               260                 265                 270
   Asp Pro Gln Met Leu Ser Glu Glu Val Arg Gln Arg Leu Gln Ala Phe
           275                 280                 285
   Arg Gln Asp Thr Tyr Leu Gln Ile Ala Ala Phe Thr Arg Ala Ile Asp
       290                 295                 300
   Gln Glu Thr Glu Glu Val Gln Gln Gln Leu Ala Pro Pro Pro Pro Gly
   305                 310                 315                 320
   His Ser Ala Phe Ala Pro Glu Phe Gln Gln Thr Asp Ser Gly Lys Val
                   325                 330                 335
   Leu Ser Lys Leu Gln Ala Arg Leu Asp Asp Leu Trp Glu Asp Ile Thr
               340                 345                 350
   His Ser Leu His Asp Gln Gly His Ser His Leu Gly Asp Pro
           355                 360                 365
<210> 2
   <211> 95
   <212> PRT
   <213> Séquence artificielle
<220>
   <223> Description de la séquence artificielle: peptide synthetique AA4RP
<400> 2
   Ala Thr Gln Ala Arg Lys Gly Phe Trp Asp Tyr Phe Ser Gln Thr Ser
    1               5                   10                  15
   Gly Asp Lys Gly Arg Val Glu Gln Ile His Gln Gln Lys Met Ala Arg
               20                  25                  30
   Glu Pro Ala Thr Leu Lys Asp Ser Leu Glu Gln Asp Leu Asn Asn Met
           35                  40                  45
   Asn Lys Phe Leu Glu Lys Leu Arg Pro Leu Ser Gly Ser Glu Ala Pro
       50                  55                  60
   Arg Leu Pro Gln Asp Pro Val Gly Met Arg Arg Gln Leu Gln Glu Glu
   65                  70                  75                  80
   Leu Glu Glu Val Lys Ala Arg Leu Gln Pro Tyr Met Ala Glu Ala
                   85                  90                  95

## Description

La présente invention concerne des compositions et méthodes pour le dosage ou la détection de « l'Apolipoprotein AIV related protein » (AA4RP). Elle concerne notamment une méthode permettant de détecter et de quantifier de façon directe l'AA4RP. Cette invention concerne également des produits synthétiques de l'AA4RP, les anticorps correspondants, les kits les comprenant, et leurs utilisations pour détecter et quantifier l'AA4RP dans un échantillon biologique.

L'augmentation des niveaux des triglycérides plasmatiques est l'un des déterminants majeurs dans le développement des maladies cardiovasculaires responsables actuellement de la majorité des décès dans le monde (Hokanson and Austin 1996). Des études ont montré que les altérations du niveau ou de la structure des apolipoprotéines sont liées à des niveaux anormaux de lipides et à une augmentation du risque d'athérosclérose (Rubin, Krauss et al. 1991; Plump, Smith et al. 1992; Duverger, Tremp et al. 1996; Huang, Liu et al. 1998; Lusis 2000; Rubin and Tall 2000). Un exemple très pertinent de l'implication des apolipoprotéines dans l'homéostasie des triglycérides plasmatiques est celui de l'AA4RP. Cette apolipoprotéine a été découverte récemment et a été décrite comme étant un facteur important dans la régulation du métabolisme des triglycérides chez la souris et chez l'homme (Pennacchio, Olivier et al. 2001). Des souris exprimant le transgène AA4RP humain montrent une diminution du niveau des triglycérides plasmatiques de 3 fois par rapport aux souris contrôles. Inversement, des souris knockout n'ayant pas le gène codant pour l'AA4RP présentent une concentration des triglycérides plasmatiques augmentée de 4 fois par rapport aux souris contrôles. Les variations du niveau des triglycérides observées chez les souris transgéniques et chez les souris knockout AA4RP vont dans le même sens que celles du niveaux de l'apolipoprotéine CIII (Apo CIII), connue comme étant un indicateur positif des niveaux des triglycérides, puisque cette apolipoprotéine inhibe l'hydrolyse des triglycérides par la lipoprotéine lipase (LpL). La concentration plasmatique de l'Apo CIII est diminuée chez les souris transgéniques AA4RP humaine après un régime riche en lipides alors que la concentration de l'Apo CIII chez les souris knockout est augmentée, indiquant que l'un des mécanismes d'action de l'AA4RP pourrait être la régulation de l'expression du gène codant pour l'Apo CIII et confirmant ainsi le rôle positif de l'AA4RP dans l'élimination des triglycérides.
Chez l'homme, il a été démontré qu'il existe une corrélation très étroite entre le polymorphisme de l'AA4RP et le niveau des triglycérides. En effet, les allèles mineurs SNPs1-3 du polymorphisme de l'AA4RP sont associés avec des niveaux élevés de triglycérides. Un résultat représentatif est l'augmentation de 30% du niveau des triglycérides chez des individus possédant l'un de ces allèles comparés aux sujets homozygotes pour l'allèles sauvage (Pennacchio, Olivier et al. 2001).

Les résultats obtenus chez la souris et chez l'homme montrent clairement l'importance du rôle de l'AA4RP dans l'homéostasie des triglycérides et ils suggèrent une possible utilisation de l'AA4RP comme marqueur dans le pronostic ou le diagnostic des hypertriglycéridémies. Il s'avère donc particulièrement pertinent de développer une méthode permettant de détecter et/ou de quantifier l'AA4RP.

Pour différentes raisons, la mesure de la concentration plasmatique de l'AA4RP est assez difficile à réaliser. En premier lieu, l'AA4RP montre 27% d'identité et 48% de similarité avec l'apolipoprotéine AIV (Apo AIV). En second lieu, l'AA4RP est présente à très faible concentration dans le plasma. Il était donc primordial de développer une méthode spécifique et sensible pour quantifier et/ ou détecter cette apolipoprotéine.

Cette protéine présente la structure primaire représentée ci-dessous, comprenant 366 acides aminés (SEQ ID NO : 1).

Cette protéine correspond à la séquence de la protéine RAP3 mentionnée antérieurement comme potentiellement impliquée dans la régénération hépatique (numéro d'accès Genbank : AF202889.1 : Van der Vliet H.N., Groenink M., Leegwater A.C.J. and Chamuleau R.A.F.M. Submitted (09-NOV-1999) Experimental Hepatology, Academic Medical Center, Meibergdreef 9, Amsterdam 1105 AZ, Netherlands). Elle correspond également à la protéine AA4RP (« Apolipoprotein AIV-Related Protein ») de la demande de brevet international WO01/00803 A2 (SEQ ID NO : 3 de cette demande).

La présente invention fournit une stratégie pour produire un peptide synthétique spécifique de l'AA4RP (qui sert à la production d'anticorps specifiques anti-AA4RP) et une nouvelle méthode immuno-enzymatique permettant de détecter et de quantifier de façon directe cette apolipoprotéine. La quantification de l'AA4RP est réalisée avantageusement par la technique ELISA (Enzyme Linked Immuno-Sorbant Assay) de type sandwich. L'anticorps anti-AA4RP est fixé sur un substrat solide pour la capture de la protéine, puis le même anticorps, cette fois-ci marqué par une enzyme, est utilisé comme anticorps de détection de l'AA4RP fixé par le premier anticorps. La phase de détection est effectuée en présence d'un substrat de l'enzyme et l'importance de la réaction développée entre l'enzyme et son substrat est directement proportionnelle à la concentration de l'AA4RP. Pour détecter, quantifier ou purifier l'AA4RP à l'aide d'anticorps spécifiques anti-AA4RP, on peut utiliser soit des anticorps polyclonaux, soit des anticorps monoclonaux ou un mélange de ces derniers.

L'invention décrit également de tels anticorps, des kits les comprenant et leurs utilisations pour détecter, quantifier ou purifier l'AA4RP dans des échantillons, notamment le sérum ou le plasma. Ces anticorps fournissent également une nouvelle approche pour moduler l'activité de l'AA4RP in vitro ou in vivo, et pour réguler le métabolisme lipidique chez un sujet.

Un premier objet particulier de l'invention concerne un peptide synthétique spécifique de l'AA4RP substantiellement pur, consistant en la séquence SEQ ID NO : 2.

Un autre objet de l'invention concerne une méthode de production d'anticorps anti-AA4RP comprenant une étape d'immunisation à l'aide d'un peptide synthétique spécifique de l'AA4RP tel que défini ci-dessus. Cette invention comprend également les anticorps préparés selon cette méthode, de même que, plus généralement, les anticorps capables de lier un peptide tel que défini ci-dessus ainsi que les fragments ou dérivés de tels anticorps.

Un autre aspect de cette invention concerne une méthode de détection ou de dosage de l'AA4RP dans des échantillons biologiques (notamment dans des lipoparticules préparées ou dans des échantillons de plasma entier ou de sérum), à l'aide d'un anticorps (incluant un fragment ou un dérivé de ce dernier) tel que défini ci-dessus.

Un autre objet de cette invention concerne une méthode de détection de la présence d'une prédisposition ou d'un risque de développer un désordre lié au métabolisme des lipides chez un sujet, comprenant la détection in vitro, dans un échantillon dudit sujet, de l'AA4RP à l'aide d'un anticorps (incluant un fragment ou un dérivé de ce dernier) tel que défini ci-dessus.

Un autre objet de cette invention concerne une méthode de détection ou de suivi chez un sujet de la capture cellulaire de lipoprotéines riches en triglycérides et des HDL, comprenant la détection in vitro de particules contenant de l'AA4RP à l'aide d'un anticorps (incluant un fragment ou un dérivé de ce dernier) tel que défini ci-dessus.

Un autre objet de l'invention concerne une méthode de détection et/ou de suivi de la formation, du développement ou de la progression de l'athérosclérose chez un sujet, comprenant la détection in vitro, dans un échantillon dudit sujet, de la quantité ou du niveau d'AA4RP à l'aide d'un anticorps (incluant un fragment ou un dérivé de ce dernier) tel que décrit ci-dessus.

Le sujet est généralement un mammifère, de préférence un être humain, de façon encore plus préférée un sujet présentant un risque de développer des maladies cardiovasculaires liées à une dyslipidémie (plus préférée une hypertriglycéridémie) comme une maladie coronarienne par exemple.

Un autre objet de cette invention concerne une composition pharmaceutique comprenant un anticorps (incluant un fragment ou un dérivé de ce dernier) tel que défini ci-dessus et un excipient ou un véhicule acceptable sur le plan pharmaceutique.

### Peptides Spécifiques

Comme indiqué ci-dessus, un aspect de la présente invention concerne un peptide synthétique spécifique de l'AA4RP substantiellement pur consistant en la séquence SEQ ID NO : 2. Le terme « substantiellement pur» indique que le peptide est essentiellement dépourvu de séquences d'acides aminés présentes dans l'Apo AIV et/ou de protéines contaminantes normalement présentes ou associées à des lipoparticules, telles que notamment l'Apo Al. Le terme « synthétique » indique que le peptide n'est pas une molécule obtenue de façon naturelle mais qu'il a été préparé grâce à un procédé artificiel (e.g., synthèse chimique, assemblage, etc.) notamment tel que décrit dans les exemples. Dans ce contexte, le peptide synthétique spécifique de l'AA4RP de l'invention est préférentiellement essentiellement non-glycosylé.

La présente invention démontre à présent que des peptides synthétiques spécifiques de l'AA4RP peuvent être produits et utilisés pour générer des anticorps anti-AA4RP spécifiques. L'invention démontre par ailleurs que de tels anticorps sont capables de se lier de façon spécifique à l'AA4RP obtenue soit de façon naturelle soit sous la forme d'un antigène soluble, soit incluse dans des lipoparticules. L'invention démontre également que de tels anticorps peuvent se lier à différentes lipoparticules contenant de l'AA4RP (VLDL et HDL). Ces peptides synthétiques spécifiques de l'AA4RP et leurs anticorps correspondants représentent ainsi de nouveaux produits particulièrement avantageux pour détecter l'AA4RP et quantifier de façon spécifique cette apolipoprotéine.

Ce fragment peptidique de 95 acides aminés correspond aux résidus 20 à 114 de la séquence de l'AA4RP humaine mature.

Comme illustré dans les exemples, ce peptide (comprenant la séquence SEQ ID NO: 2) peut être préparé de façon particulièrement avantageuse par synthèse en phase solide, plus particulièrement en utilisant une stratégie Boc/Bzl (Merrifield 1963).

Le terme « dérivé » inclut des peptides comprenant une ou plusieurs mutations, substitutions, délétions et/ou additions d'un ou de plusieurs résidus d'acides aminés et présentant substantiellement la même spécificité antigénique. Des exemples typiques de dérivés incluent des variations de séquence dues au polymorphisme de l'AA4RP, à l'épissage, etc.. Des dérivés particulièrement divulgés comprennent une séquence SEQ ID NO : 2 ou une séquence modifiée comprenant au plus 5 résidus d'acides aminés distincts de ceux présents dans la SEQ ID NO: 2. Les résidus additionnels peuvent correspondre à des résidus de séquence de transport ou de liaison, à des groupes protecteurs, etc.. De plus, le peptide peut être modifié par exemple, par voie chimique, physique et/ou enzymatique, de manière à augmenter sa stabilité, augmenter son immunogénicité ou encore incorporer un « Tag » ou toute autre molécule chariot ou un marqueur, etc.. Des exemples de telles modifications incluent une glycosylation, l'addition d'un chariot, d'un marqueur (e.g., marquage radioactif ou enzymatique, etc.), etc..

Le terme « fragment » désigne tout peptide comprenant de 5 à 95 résidus consécutifs de la séquence SEQ ID NO : 2, de préférence de 10 à 95. Le terme « fragment » inclut toute portion de la séquence SEQ ID NO : 2 comprenant un épitope.

Un objet particulier de l'invention concerne un peptide synthétique spécifique de l'AA4RP caractérisé en ce qu'il comprend des épitopes spécifiques de l'AA4RP et en ce qu'il est dépourvu d'épitopes spécifiques de l'Apo AIV, et en ce qu'il consiste en la séquence SEQ ID NO : 2.

Les peptides peuvent être solubles, purifiés ou complexés à une molécule porteuse, telle que KLH (Keyhole Limpet Hemocyanin) ou la sérum albumine par exemple, ou encore toute autre molécule inerte (e.g., synthétique) telle qu'une bille, etc.. Selon un mode particulier de réalisation de l'invention, les peptides sont couplés à une molécule porteuse. Ils le sont en particulier dans le cadre de leur utilisation pour produire des anticorps. Le couplage peut être réalisé selon des méthodes conventionnelles connues de l'homme du métier (Vaitukaitis, Robbins et al. 1971) (Bassiri 1979).

Les peptides peuvent également être conjugués ou couplés à une molécule polypeptidique hétérologue, telle qu'une molécule biologiquement active par exemple. Le caractère hétérologue désigne tout peptide qui n'est pas originaire d'une molécule AA4RP.

Un objet particulier de l'invention concerne une composition caractérisée en ce qu'elle comprend un peptide synthétique consistant en la séquence SEQ ID NO : 2, et en ce qu'elle est dépourvue d'autres protéines et en particulier des apolipoprotéines.

Les peptides peuvent être utilisés dans le cadre de méthodes de criblage pour des essais de titration, comme contrôles, standards ou pour calibrer des essais. Ils peuvent également être utilisés pour moduler l'activité de l'AA4RP. Ils sont également particulièrement utiles pour produire des anticorps anti-AA4RP.

### Anticorps

A cet égard, un autre objet de l'invention concerne tout anticorps capable de se lier à un peptide tel que défini ci-dessus. L'anticorps peut être polyclonal ou monoclonal. De plus, le terme anticorps inclut également tous fragments et dérivés d'anticorps, en particulier les fragments et dérivés desdits anticorps monoclonaux ou polyclonaux présentant substantiellement la même spécificité antigénique. Ces derniers comprennent des fragments d'anticorps (e.g., Fab, F(ab')2, CDRs, etc.), des anticorps humanisés, des anticorps polyfonctionnels, des anticorps monocaténaires (ScFv), etc.. Les anticorps de l'invention peuvent être produits à l'aide de méthodes conventionnelles, comprenant l'immunisation d'un animal et la récupération de son sérum (polyclonal) ou de cellules spléniques (de manière à produire des hybridomes par fusion avec des lignées cellulaires appropriées).

Des méthodes de production d'anticorps polyclonaux à partir d'espèces variées incluant les souris, les rongeurs, les primates, les chevaux, les cochons, les lapins, la volaille, etc. sont décrites par exemple dans Vaitukaitis et al. (Vaitukaitis, Robbins et al. 1971). L'antigène est combiné avec un adjuvant (e.g., adjuvant de Freund) et administré à un animal, typiquement par injection sous-cutanée. Des injections répétées peuvent être réalisées. Les échantillons sanguins sont collectés et l'immunoglobuline ou le sérum sont séparés.

Des méthodes de production d'anticorps monoclonaux à partir de différentes espèces peuvent être trouvées par exemple dans Harlow et al. (Harlow 1988) ou dans Kohler et al. (Kohler and Milstein 1975). Ces méthodes comprennent l'immunisation d'un animal avec un antigène, suivie de la récupération des cellules spléniques qui sont ensuite fusionnées avec des cellules immortalisées, telles que des cellules de myélome. Les hybridomes résultant produisent des anticorps monoclonaux et peuvent être sélectionnés par dilutions limites de manière à isoler les clones individuels. Les anticorps peuvent également être produits par sélection de banques combinatoires d'immunoglobulines, telles que celles divulguées par exemple dans Ward et al. (Ward, Gussow et al. 1989).

Des anticorps préférés de l'invention sont préparés par immunisation à l'aide d'un peptide synthétique spécifique de l'AA4RP substantiellement pur tel que décrit ci-dessus, consistant en la séquence SEQ ID NO : 2,

Les fragments Fab ou F(ab')2 peuvent être produits par digestion à l'aide d'une protéase selon les techniques conventionnelles. Les anticorps humanisés peuvent être préparés selon l'une des méthodes décrites, par exemple, dans Riechmann et al. (Riechmann 1988) (Jones 1986).

L'invention concerne également une méthode de production d'anticorps anti-AA4RP, comprenant l'injection d'un peptide de séquence SEQ ID NO : 2 et la récupération des anticorps ou des cellules productrices d'anticorps. La méthode permet avantageusement la production d'anticorps spécifiques. La spécificité peut être vérifiée par la démonstration de l'absence d'une réaction croisée avec d'autres protéines circulantes sanguines. Plus généralement, la spécificité indique que les anticorps sont capables de se lier à l'AA4RP avec une affinité supérieure à tout autre antigène. Comme illustré dans les exemples, les anticorps polyclonaux de la présente invention peuvent être utilisés pour détecter et/ou doser l'AA4RP avec une grande efficacité.

Les anticorps peuvent être couplés à des fragments hétérologues tels que des toxines, des marqueurs, des médicaments ou tout autre agent thérapeutique, de façon covalente ou non, soit directement, soit par l'intermédiaire d'agents de couplage. Les marqueurs peuvent être choisis parmi les radio-marqueurs, des enzymes, des agents fluorescents, des particules magnétiques, etc.. Des toxines préférées sont par exemple la toxine diphtérique, la toxine botulique, etc.. Les médicaments ou les agent thérapeutiques sont choisis notamment parmi des lymphokines, des antibiotiques, des séquences anti-sens, des facteurs de croissance, etc.. Des méthodes permettant de réaliser le couplage des anticorps et de tels fragments hétérologues sont illustrées par exemple dans le brevet US 4,277,149 et dans le brevet US 3,996,345.

Les anticorps de l'invention possèdent de nombreuses applications incluant des applications thérapeutiques, prophylactiques, diagnostiques, de purification, de détection, etc..

*In vitro,* ils peuvent être utilisés comme agents de criblage ou pour purifier des antigènes provenant d'échantillons divers, incluant des échantillons biologiques variés (e.g., des échantillons sanguins). Ils peuvent également être utilisés pour détecter ou quantifier la présence ou la quantité d'AA4RP dans les lipoparticules au sein d'un échantillon collecté à partir d'un sujet, typiquement, un échantillon sanguin provenant d'un mammifère ou, de manière préférée, d'un être humain.

### Détection / Dosage

A cet égard, un autre objet de l'invention concerne une méthode de détection, mesure, dosage ou quantification de l'AA4RP au sein d'un échantillon biologique, comprenant la mise en contact de l'échantillon avec un anticorps tel que défini ci-dessus (incluant des fragments ou des dérivés de ce dernier) et la détection, mesure, dosage ou quantification (de la présence) de complexes immuns antigène-anticorps. Typiquement cette méthode permet la détermination des niveaux d'AA4RP dans un échantillon, par comparaison à des conditions standards ou à une courbe de calibration par exemple.

La détermination des complexes immuns peut être réalisée à l'aide de techniques conventionnelles telles que des méthodes immunologiques directes ou compétitives, par exemple la technique RIA (Radio Immuno Assay), EIA (Electro Immuno Assay), par néphélométrie, turbidimétrie, immunoblot quantitatif, calorimétrie, interférométrie, résonance de surface, mesure de champ de force, autres biosenseurs en développement, etc.. Cependant, un objet particulièrement préféré de l'invention concerne une méthode impliquant la technique ELISA de type sandwich. En effet, comme indiqué ci-dessus, les anticorps sont spécifiques et peuvent reconnaître l'AA4RP dans un échantillon permettant ainsi de doser de façon précise, sensible et efficace l'AA4RP avec un anticorps polyclonal (ou un mélange d'anticorps monoclonaux) anti-AA4RP.

Un objet plus préféré de l'invention concerne donc une méthode de détection, mesure, dosage ou quantification de l'AA4RP au sein d'un échantillon biologique, comprenant la mise en contact de l'échantillon (ou de dilutions de celui-ci) avec un premier anticorps immobilisé sur un support, dit anticorps de capture, cet anticorps étant tel que défini ci-dessus (incluant des fragments ou des dérivés de ce dernier), et la révélation des complexes immuns antigène-anticorps éventuellement formés au moyen d'un deuxième anticorps marqué, dit anticorps de révélation, cet anticorps étant tel que défini ci-dessus (incluant des fragments ou des dérivés de ce dernier).

Dans un mode particulièrement préféré, l'anticorps de capture et l'anticorps de révélation sont des anticorps polyclonaux. Ils peut s'agir du même anticorps polyclonal. L'anticorps de capture peut être immobilisé sur tout type de support, notamment plastique, tel qu'une plaque, bille, colonne, gel, etc.. Il s'agit avantageusement d'une plaque multi-puits. L'immobilisation est typiquement réalisée par adsorption des anticorps sur la surface des puits. Il est entendu que toute autre technique de dépôt peut être mise en oeuvre, directe ou indirecte, covalente ou non. L'anticorps de capture est utilisé typiquement sous forme soluble. Il est préalablement marqué pour permettre sa détection et sa quantification. Le marquage peut être de nature variée, comme un marquage radioactif, fluorescent, enzymatique, luminescent, etc.. Dans un mode particulier de mise en oeuvre, le marquage est un marquage enzymatique, et les complexes immuns sont révélés par mesure de l'activité enzymatique. Un exemple spécifique de marquage enzymatique est la peroxydase, dont l'activité peut être révélée en présence du substrat OPD par exemple.

Un objet particulièrement préféré de l'invention concerne une méthode de détection, mesure, dosage ou quantification de l'AA4RP au sein d'un échantillon biologique, comprenant la mise en contact de l'échantillon (ou de dilutions de celui-ci) avec un support sur lequel est immobilisé un anticorps de capture polyclonal tel que défini ci-dessus, dans des conditions propices à la réalisation de complexes immuns spécifiques, et la révélation des complexes immuns antigène-anticorps éventuellement formés au moyen d'un anticorps de révélation polyclonal marqué, cet anticorps polyclonal étant tel que défini ci-dessus.

La méthode peut être réalisée sur différents échantillons biologiques, incluant du plasma, du sérum, du fluide interstitiel, du surnageant de cultures cellulaires, etc.. L'échantillon peut être collecté chez un sujet (e.g., un sujet humain) et utilisé directement pour l'essai. De manière alternative, l'échantillon peut être dilué et/ou stocké (par exemple sous forme congelée) pour être testé plus tard. L'invention permet également la mesure des concentrations d'AA4RP contenue dans les lipoparticules grâce à l'utilisation des anticorps anti-AA4RP spécifiques, avec une sensibilité, une reproductibilité et une répétabilité très élevées.

La détection peut être réalisée dans différentes conditions expérimentales, cliniques, épidémiologiques, pronostiques et diagnostiques. En particulier, la méthode peut être utilisée pour détecter la prédisposition de certains individus à développer des désordres liés au métabolisme des lipides.

Un objet particulier de l'invention concerne ainsi une méthode de détection de la présence d'une prédisposition ou d'un risque de développer un désordre lié au métabolisme des lipides chez un sujet, comprenant la détection *in vitro* ou la mesure *in vitro,* à partir d'un échantillon prélevé sur le sujet, de lipoparticules comprenant l'AA4RP, à l'aide d'un anticorps tel que défini ci-dessus (incluant des fragments ou des dérivés de ce dernier). Les niveaux d'AA4RP (par comparaison à une valeur moyenne chez les sujets normaux) peuvent être caractéristiques du risque élevé de développer des désordres liés au métabolisme des lipides.

Un autre objet de l'invention concerne une méthode de détection ou de suivi de la capture cellulaire des HDL et des lipoprotéines riches en triglycérides chez un sujet, comprenant la détection *in vitro* des quantités d'AA4RP présentes dans des lipoparticules, à l'aide d'un anticorps tel que défini ci-dessus (incluant des fragments ou des dérivés de ce dernier).

Un autre objet de l'invention concerne une méthode de suivi d'un traitement destiné à corriger des désordres liés au métabolisme des lipides chez un sujet, comprenant la détection des niveaux d'AA4RP *in vitro,* dans un échantillon provenant dudit sujet, à l'aide d'un anticorps tel que défini ci-dessus (incluant des fragments ou des dérivés de ce dernier), après l'administration dudit traitement audit sujet. L'efficacité du traitement est corrélée au niveau d'AA4RP chez le sujet. Ces résultats peuvent être corrélés avec la capacité du traitement à réguler les niveaux ou l'activité de l'AA4RP ou encore la capacité à restaurer une activité ou un niveau normal de l'AA4RP chez un sujet.

Un autre objet de l'invention concerne une méthode d'évaluation de l'état physiologique d'un sujet, e.g. le niveau du métabolisme des lipides chez un sujet, comprenant la détection des niveaux de l'AA4RP *in vitro ou ex vivo* dans un échantillon provenant dudit sujet, à l'aide d'anticorps tels que définis ci-dessus (incluant les fragments et les dérivés de ce dernier).

Comme indiqué ci-dessus, ces méthodes peuvent être réalisées sur différents échantillons (typiquement du plasma ou du sérum) et par RIA, EIA, néphélométrie, turbidimétrie immuonblot quantitatif, calorimétrie, interférométrie, résonance de surface, mesure de champ de force, autres biosenseurs en développement, etc..., ou de préférence grâce à un essai ELISA de type sandwich.

L'invention comprend en outre une composition pharmaceutique comprenant un anticorps tel que décrit ci-dessus et éventuellement un excipient ou un véhicule acceptable sur le plan pharmaceutique.

Cette invention concerne également un kit comprenant un peptide ou un anticorps tel que décrit ci-dessus. Le kit peut être utilisé pour détecter ou quantifier l'AA4RP dans tout échantillon.

D'autres aspects et avantages de l'invention seront décrits dans les exemples qui suivent, qui doivent être considérés comme illustratifs et ne limitent pas l'étendue de l'invention.

### Légende des figures

Figure 1 :
   Spécificité des anticorps.
   Contrôle en SDS PAGE de la spécificité des anticorps anti-AA4RP et mise en évidence de la distribution de l'AA4RP dans les VLDL et les HDL.
Figure 2:
   Essai ELISA sur l'AA4RP
   La Courbe de calibration est construite à partir des DO en fonction des concentrations du peptide 20-114 (◆), d'un plasma de sujets normolipidique (π) et des HDL préparées à partir de plasma de sujet normolipidique (●) selon le protocole indiqué dans l'exemple 3.
Figure 3 :
   Concentrations plasmatiques de l'AA4RP calculées à partir d'une gamme peptide 20-114 ou d'un pool de plasma de sujets normolipidiques selon méthode ELISA
Figure 4 :
   Dosage ELISA de l'AA4RP chez des souris C57 BL/6 des souris FVB non transgéniques, des souris knock-out (KO) AA4RP à fonds génétique FVB et des souris transgéniques pour l'AA4RP humaine (fonds génétique FVB).

### EXEMPLES

### Exemple 1 : Séquence peptidique

### 1. Choix de la séquence peptidique appropriée

Le fragment peptidique suivant a été synthétisé. Ce fragment a été déterminé en utilisant différents algorithmes permettant de prédire la flexibilité, l'hydrophilicité, l'antigénicité, et les structures secondaires. Ce fragment de 95 acides aminés correspond aux résidus 20 à 114 de la séquence SEQ ID NO : 1.

### 2. Synthèse peptidique.

Le peptide a été synthétisé par la méthode de synthèse en phase solide (Merrifield 1963) sur un synthétiseur automatique modèle ABI 431 A (Applied Biosystems Inc., Californie, USA) en utilisant une stratégie Boc/Bzl sur une résine MBHA 0,5 mmol (0,57 mmol/g). Chaque acide aminé a été couplé deux fois en présence de dicyclohexylcarbodiimide/hydroxybenzotriazole sans capping. Les groupes protecteurs de chaîne latérale sont les suivants : Arg(Ts), Asp(Ochex), Glu(Ochex), Lys(2-Cl-Z), His(Dnp), Ser(Bzl), Thr(Bzl), Met(O), Trp(formy) et Tyr(Br-Z).

Le groupe Dnp sur le résidu Histidine a été éliminé du peptide avant clivage à partir du support par traitement en présence de β-mercaptoéthanol 10%, diisopropyléthylamine 5% dans un milieu DCM pendant 2 heures puis dans un milieu NMP pendant 2 heures. La résine peptidyle a été traitée avec du TFA 50% dans un milieu DCM pendant 20 minutes pour éliminer l'acide aminé terminal Boc. Le peptide a été clivé de la résine et déprotégé simultanément selon une procédure à HF lente et rapide : la résine (1 g) a été traitée avec du HF anhydre (2,5 ml) en présente de p-crésol (0,75 g), p-thiocrésol (0,25 g) et de diméthylsulfure (0,5 ml) à 0°C.

3 heures après, le fluorure d'hydrogène et le diméthylsulfure ont été éliminés par évaporation sous vide et les scavengers résiduels et les produits secondaires ont été extraits avec de l'éther diéthylique. Les appareils de la réaction ont été rechargés avec du p-crésol (0,75 g), du p-thiocrésol (0,25 g) et 10 ml de HF anhydre puis le mélange a été laissé en incubation à 0°C pendant 1 heure et demie. Le fluorure d'hydrogène a été éliminé par évaporation et le résidu a été mélangé en présence d'éther diéthylique. Le résidu a été filtré, lavé avec de l'éther diéthylique et extrait avec 200 ml d'une solution d'acide acétique aqueuse à 10% puis lyophilisé.

### 3. Spectrométrie de masse

La masse moléculaire a été déterminée en utilisant un spectromètre de masse à électrospray d'ions. Le spectre de l'électrospray a été obtenu en utilisant un appareil API (Perkin-Elmer-Sciex) sur un spectromètre de masse par électrospray d'ions à quadrupole simple, équipé avec un spray d'ions (électrospray assisté d'un nébuliseur) (Sciex, Toronto, Canada).

### 4. Immunisation

Le peptide a été émulsifié dans l'adjuvant complet de Freund et injecté par voie sous-cutanée à des lapins en utilisant 0,5 mg de peptide par injection pour les deux premières injections, suivi à 15 jours d'intervalle d'injections de rappel dans le même adjuvant mais en utilisant 0,25 mg de peptide seulement.

### Exemple 2: Isolement et caractérisation des anticorps de lapin anti-AA4RP spécifiques.

### 1. Isolement des anticorps anti-AA4RP

Les anticorps polyclonaux ont été isolés par précipitation avec 27% de sulfate de sodium puis purifiés par chromatographie d'affinité sur gel de sépharose 4B activé (pharmacia, Uppsala, Suède), couplé avec le peptide de l'AA4RP résidu 20 à 114 AA (Axen, Porath et al. 1967). Les protéines non retenues sur le gel antigènique ont été éliminées par lavage avec une solution saline tamponnée phosphate (PBS : Phospate 50 mmol/L, pH 7,2, NaCl 150 mmol/L). Les fractions non liées de manière spécifique sur le gel AA4RP ont été éliminées par 25 mmol/L de PBS. L'élution des IgG polyclonales spécifiques de l'AA4RP a été accomplie en utilisant la glycine 0,2 M à pH 2,8. Les anticorps purifiés ont été dialysés immédiatement contre 10 mmol/L de PBS puis concentrés par ultrafiltration en utilisant un système Amicon (seuil de coupure 100 kD) (Amicon, Bervely, USA), testés en terme de contenu en protéine (Lowry O.H. 1951), puis conservés par aliquotes de 1 ml (1 mg) à - 30°C.

### 2. Analyse en Western Blot

### 2.1- Protocole :

La pureté et la spécificité de l'anticorps ont été testées en Western Blot (Towbin, Staehelin et al. 1979). Des particules VLDL, LDL et HDL humaines ont été soumises à une électrophorèse dénaturante sur gel SDS-PAGE (5 à 24%) puis transférées sur une membrane de nitrocellulose et mises à réagir avec l'anticorps anti-AA4RP humaine purifié. Les protéines immunoréactives ont été visualisées avec un anticorps polyclonal anti-IgG conjugué à la peroxydase de raifort (Sanofi-Diagnostics Pasteur, Mares-la-Coquette, France). Le développement de la réaction est effectué par chemiluminescence (Amersham, Pharmacia, Biotec).

### 2.2- Résultats

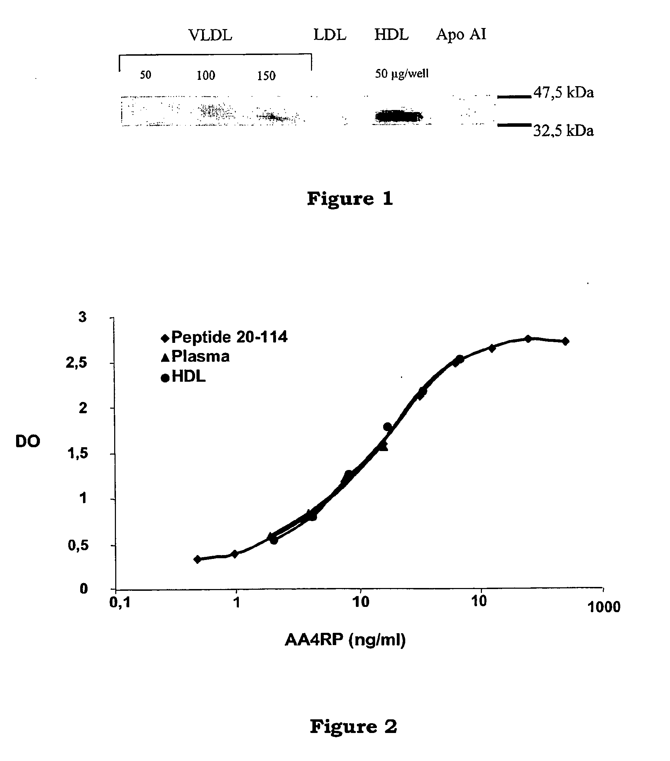

Les résultats obtenus sont présentés sur la Figure 1. Il apparaît sur cette figure que la bande spécifique qui est révélée par l'anticorps anti-AA4RP est située entre les marqueurs de poids moléculaire 32,5 et 47,5 kDa.

### 2.3- Interprétation

Le résultat de l'immunoblot sur les différentes lipoprotéines humaines, montrent une localisation de l'AA4RP au niveau des VLDL et des HDL, avec une distribution très majoritaire au niveau des HDL.

La présence de l'AA4RP au niveau des VLDL expliquerait le rôle de cette apolipoprotéine dans la régulation du métabolisme de ces lipoprotéines riche en triglycérides, et donc la modulation de la concentration de ces lipides athérogènes.

La localisation de l'AA4RP au niveau des HDL est certainement en relation avec le rôle de ces particules dans le transport inverse du cholestérol. En effet la plupart des apolipoprotéines localisées au niveau des HDL sont des promoteurs de la captation du cholestérol des cellules périphériques par les HDL et de son retour vers le foie où il est éliminé.

### Exemple 3 : Dosage ELISA de l'AA4RP (type sandwich)

### 1. Réactifs et matériels utilisés dans le cadre du test ELISA

### 1.1- Anticorps de capture

La solution mère de l'anti-AA4RP est concentrée à 1 mg/ml, le coating (fixation de l'anticorps dans les puits d'une plaque 96 puits) est effectué à 10 µg/ml, la dilution de l'anticorps est realisée dans une solution phosphate saline (PBS 0,1M, NaCl 0,15 M, pH 7,2 à 7,4).

### 1.2- Anticorps de détection

Le même anticorps utilisé pour la capture est marqué à la peroxydase. Il est dilué au 1/5000 dans le tampon PBS 0,1 M contenant de la BSA à 1%.

### 1.3- Standard

Le peptide 20-114 est utilisé pour la construction de la gamme standard. La concentration du peptide est de 1 mg/ml.
Pour la construction d'une courbe de calibration, le standard doit être dilué dans une solution de PBS 0,1 M/BSA 1 % selon le tableau ci-dessous :

| **Points** | **Concentration (ng/ml)** |
|---|---|
| 1 | 125,00 |
| 2 | 62,50 |
| 3 | 31,25 |
| 4 | 15,62 |
| 5 | 7,81 |
| 6 | 3,90 |
| 7 | 1,95 |
| 8 | 0,97 |
| 9 | 0,48 |
| 10 | 0,24 |
| 11 | 0,12 |

### 1.4- Préparation des échantillons

Les échantillons frais sont recommandés pour l'analyse. Les plasmas doivent être collectés grâce à des procédures établies et utilisées dans les laboratoires cliniques. Si besoin, le plasma peut être stocké entre 2 et 8°C jusqu'à une semaine. Les échantillons conservés congelés peuvent être utilisés pendant une plus longue période.
Les échantillons sont dilués selon leur origine :
- Plasma de sujets normolipidiques : de 1,3 à 4 fois.
- Plasma de sujets hypertriglycéridémiques : de 2 à 16 fois.
- HDL préparées à partir de plasma par ultracentrifugation : de 4 à 128 fois.

Les dilutions sont effectuées dans une solution PBS/BSA 1%.

### 2. Protocole

- « Coating » : la solution d'anticorps de capture anti-AA4RP non marqué est incubée une nuit à température ambiante à raison de 100 µl/puits (plaque de microtitartion de 96 puits).
- Lavage de la plaque : les anticorps non fixés sur la plaque sont éliminés par 4 lavages de la plaque de microtitration avec du PBS 0,1 M.
- Dépôt des échantillons et de la gamme standard : Les préparations de la gamme de peptide, des plasma humains ou des HDL sont déposées à raison de 100 µl par puits, puis sont incubées à 37°C pendant 2 heures.
- Lavage : l'excès du peptide, de l'AA4RP des échantillons biologiques qui n'est pas fixé par l'anti-AA4RP de capture est éliminé par 4 lavages successives de la plaque de microtitration.
- Détection : la détection est effectuée par l'anti-AA4RP marqué à la peroxydase dilué au 1/5000 dans du PBS/BSA 1%. 100 µl de la préparation est déposé par puits de la plaque de microtitration. L'incubation est effectuée à 37°C pendant 2 heures.
- Lavage : l'excès de l'anticorps anti-AA4RP de détection est éliminé par 4 lavages successives de la plaque par du PBS 0,1 M.
- Développement de la réaction enzymo-colorimétrique : réalisée par ajout de 100 µl de substrat *o*-Phénylénediamine dihydrochloride (OPD) par puits, ensuite la réaction est développée pendant 30 minutes à l'obscurité.
- Arrêt de la réaction : par ajout de 100 µl d'HCl 1N par puits.
- Lecture de l'absorbance : est effectuée par un spectrophotomètre à la longueur d'onde 492 nm.

### 3. Résultats et interprétations

### 3.1- Courbe de calibration

La courbe de calibration est construite selon une fonction à quatre inconnues représentant la croissance de la densité optique en fonction de la concentration des différentes dilutions de la gamme standard. Elle montre une allure sigmoïde caractéristique du dosage immuno-enzymatique ELISA (Figure 2).

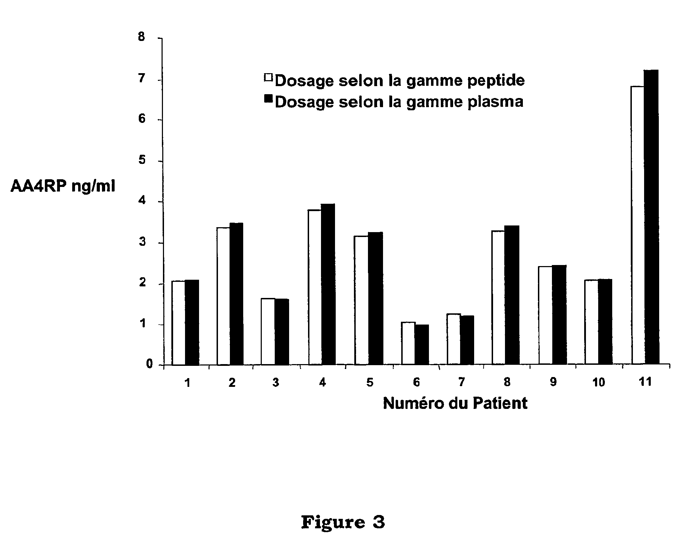

Les courbes représentatives des différentes dilutions du peptide 20-114, du plasma normolipidique et des HDL humaines sont confondues, indiquant que l'anti-AA4RP reconnaît avec la même affinité le peptide synthétique et l'AA4RP native dans un plasma ou dans des lipoprotéines humaines. En plus, le dosage de l'AA4RP chez plusieurs sujets utilisant une gamme de calibration construite soit à partir du peptide soit à partir d'un pool de plasmas humains, donne des résultats de la concentration de l'AA4RP significativement similaires (Figure 3).

### 3.2- Intervalle de travail et seuil de détection

Il est d'environ 2 Log, entre 1 et 62 ng/ml, ce qui permet de doser des échantillons dont les concentrations en AA4RP sont très variables. Le seuil de détection est d'environ 1 ng/ml, ce qui indique que le dosage est d'une très grande sensibilité.

### 3.3- Valeurs normales

Le dosage de l'AA4RP dans une population de sujets normolipidiques dont le taux des triglycérides ne dépasse pas 1 mg/ml donne des valeurs de la concentration de l'apolipoprotéine entre 8,94 et 29,44 ng/ml.

### Exemple 4 : Corrélation entre la concentration de l'AA4RP des différents compartiments plasmatique et le niveaux des triglycérides totaux

### 1. Protocole

Les corrélations des niveaux triglycérides plasmatiques avec les niveaux de l'AA4RP plasmatique ou de l'AA4RP dans les lipoprotéines non HDL (VLDL, IDL et LDL) ou de l'AA4RP des lipoprotéines HDL sont réalisées chez une population dont les taux des triglycérides sont compris entre 0,5 et 4 mg/ml.

### 1.1- Préparation des lipoprotéines HDL et non HDL

Les lipoprotéine HDL et non HDL sont préparées par précipitation de ces dernières par addition d'acide phosphotungstique et de Mg2+ aux plasmas des différents sujets (précipitation sélective des lipoprotéines contenant l'Apo B, soit les VLDL, IDL et LDL)(Burstein, Scholnick et al. 1970; Lopes-Virella, Stone et al. 1977). Brièvement, le plasma est incubé pendant 10 minutes à température ambiante avec une solution de phosphotungstate, puis centrifugé à 2500 rpm pendant 15 minutes et enfin le surnageant contenant les HDL est séparé du culot contenant les lipoprotéines non HDL.

### 1.2- Dosage des triglycérides

Les triglycérides plasmatique sont dosées par la méthode enzymo-colorémètrique, contre une gamme standard, préparée grâce au calibrateur des lipides CFAS Réf. N° 759350 (Boehringer Mannheim GmbH, Allemagne). La gamme standard a été construite de 16 à 500 µg/ml. 100 µl de chaque dilution d'échantillon ou de gamme étalon sont déposés par puits d'une plaque de titration (96 puits). Ensuite 200 µl de réactifs triglycérides Réf. 701912 (Boehringer Mannheim GmbH, Allemagne) sont rajoutés dans chaque puits, et l'ensemble de la plaque est incubé pendant 30 min. à 37°C. La lecture des Densités Optiques (DO) est effectuée à 492 nm sur le spectrophotomètre. Les concentrations en triglycérides de chaque échantillon sont calculées après construction de la courbe étalon selon une fonction linéaire y=ax+b, où y représente les DO et x les concentrations en triglycérides.

### 1.3- Dosage de l'AA4RP totales, des lipoprotéines HDL et des lipoprotéines non HDL :

Les concentrations de l'AA4RP totales du plasma et celle des lipoprotéines HDL sont dosées selon le protocole de l'exemple 3. La concentration de l'AA4RP des lipoprotéines non HDL est calculée par différence entre la concentration de l'AA4RP totale plasmatique et celle des lipoprotéines HDL.

### 2.- Résultats et interprétations :

Nous n'avons pas observé de corrélation entre les niveaux plasmatiques de l'AA4RP est ceux des triglycérides totaux ou ceux des triglycérides des lipoprotéines non HDL. Par contre, il existe une corrélation négative significative entre la concentration de la protéine et celle des triglycérides des lipoprotéines HDL (r=0,5, p<0,001). Ceci indique que plus le niveau des triglycérides augmente, plus la quantité de l'AA4RP dans les HDL diminue, confirmant ainsi le rôle anti-athèrogène des HDL et par conséquent celui de l'AA4RP présente dans ce type de lipoprotéines.

### Exemple 5 : Quantification de l'AA4RP chez des souris transgéniques pour l'AA4RP humaines

### 1.- Protocole :

Le niveau de l'AA4RP a été dosé chez des souris C57BL/6 normales, des souris FVB non transgéniques, des souris knock-out (KO) AA4RP à fonds génétique FVB et des souris transgéniques pour l'AA4RP humaine (fonds génétique FVB). Le protocole de dosage est identique à celui décrit dans l'exemple 3.

### 2.- Résultats et interprétations :

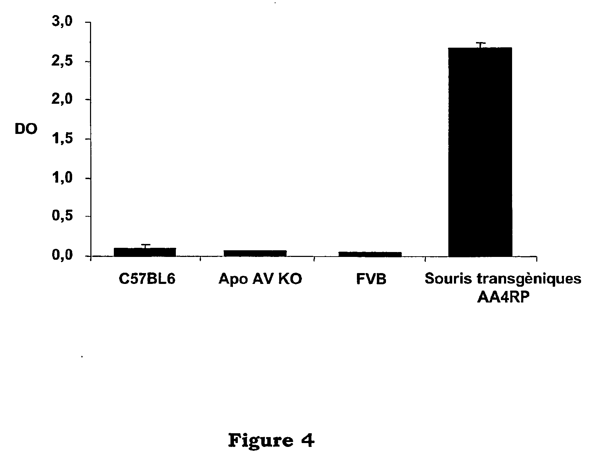

La figure 4 montre que notre dosage détecte l'AA4RP uniquement dans les souris transgéniques AA4RP humaine indiquant la spécificité de la méthode entre l'humain et la souris. Ceci est très intéressant, puisqu'il est possible de quantifier l'AA4RP exogène dans des modèles animaux modifiés sans contamination par celle endogène, ce qui permet de dissocier entre l'effet de l'AA4RP propre de l'animal et celle d'origine exogène.

### REFERENCES

Axen, R., J. Porath, et al. (1967). "Chemical coupling of peptides and proteins to polysaccharides by means of cyanogen halides." Nature **214**(95): 1302-4.
Bassiri, R. M., Dvorak J. and Utiger R.D. (1979). Thyrotrpin-releasing hormone. Methods of hormone radioimmunoassay. B. M. In: Jaffe, and Behrman H.R. (Eds). New York, New York Academic Press: p: 46.
Burstein, M., H. R. Scholnick, et al. (1970). "Rapid method for the isolation of lipoproteins from human serum by précipitation with polyanions." J Lipid Res **11**(6): 583-95.
Duverger, N., G. Tremp, et al. (1996). "Protection against atherogenesis in mice mediated by human apolipoprotein A-IV." Science **273**(5277): 966-8.
Harlow, E. a. L., D., Ed. (1988). Antibodies: a Laboratory Manual. New York, Cold Spring Harbor Laboratory Publications.
Hokanson, J. E. and M. A. Austin (1996). "Plasma triglyceride level is a risk factor for cardiovascular disease independent of high-density lipoprotein cholesterol level: a meta- analysis of population-based prospective studies." J Cardiovasc Risk **3**(2): 213-9.
Huang, Y., X. Q. Liu, et al. (1998). "Overexpression and accumulation of apolipoprotein E as a cause of hypertriglyceridemia." J Biol Chem **273**(41): 26388-93.
Jones, P. T., Dear P. H., Foote J., Neuberger M. S. and Winter G. (1986). "Replacing the complementarity-determining regions in a human antibody with those from a mouse." Nature **321**: 522-525.
Kohler, G. and C. Milstein (1975). "Continuous cultures of fused cells secreting antibody of predefined specificity." Nature **256**(5517): 495-7.
Lopes-Virella, M. F., P. Stone, et al. (1977). "Cholesterol determination in high-density lipoproteins separated by three different methods." Clin Chem **23**(5): 882-4.
Lowry O.H., R. J., Farr A.L., and Randall R.J. (1951). "Protein measurement with folin phenol reagent." J. Biol. Chem. **193**: 265-275.
Lusis, A. J. (2000). "Atherosclerosis." Nature **407**(6801): 233-41.
Merrifield, R. B. (1963). "Solide phase peptide synthesis. The synthesis of a tetrapeptide." J. Am. Chem. Soc **85**: 2149-2154.
Pennacchio, L. A., M. Olivier, et al. (2001). "An apolipoprotein influencing triglycerides in humans and mice revealed by comparative sequencing." Science **294**(5540): 169-73.
Plump, A. S., J. D. Smith, et al. (1992). "Severe hypercholesterolemia and atherosclerosis in apolipoprotein E- deficient mice created by homologous recombination in ES cells." Cell **71**(2): 343-53.
Riechmann, L., Clark M., Waldmann H. and Winter G. (1988). "Monoclonal antibody therapeutic trials in seven patients with T-cell lymphoma." Nature **332**: 323-327.
Rubin, E. M., R. M. Krauss, et al. (1991). "Inhibition of early atherogenesis in transgenic mice by human apolipoprotein Al." Nature **353**(6341): 265-7.
Rubin, E. M. and A. Tall (2000). "Perspectives for vascular genomics." Nature **407**(6801): 265-9.
Towbin, H., T. Staehelin, et al. (1979). "Electrophoretic transfer of proteins from polyacrylamide gels to nitrocellulose sheets: procedure and some applications." Proc Natl Acad Sci U S A **76**(9): 4350-4.
Vaitukaitis, J., J. B. Robbins, et al. (1971). "A method for producing specific antisera with small doses of immunogen." J Clin Endocrinol Metab **33**(6): 988-91.
Ward, E. S., D. Gussow, et al. (1989). "Binding activities of a repertoire of single immunoglobulin variable domains secreted from Escherichia coli." Nature **341**(6242): 544-6.

## Revendications

1. Peptide synthétique substantiellement pur, **caractérisé en ce qu'**il consiste en la séquence d'acides aminés SEQ ID NO : 2.

2. Peptide selon la revendication 1, **caractérisé en ce que** le peptide est soluble ou complexé à une molécule porteuse telle que KLH, la sérum-albumine ou une bille.

3. Anticorps spécifique d'un peptide selon l'une des revendications 1 ou 2, ou fragment ou dérivé dudit anticorps présentant substantiellement la même spécificité antigénique.

4. Anticorps selon la revendication 3, **caractérisé en ce qu'**il est produit par immunisation d'un animal non-humain avec un peptide selon l'une des revendications 1 ou 2, et récupération des anticorps ou des cellules productrices des anticorps.

5. Anticorps selon l'une des revendications 3 ou 4, **caractérisé en ce qu'**il s'agit d'un anticorps polyclonal.

6. Anticorps selon l'une des revendications 3 ou 4, **caractérisé en ce qu'**il s'agit d'un anticorps monoclonal.

7. Méthode de production d'un anticorps anti-AA4RP spécifique, comprenant l'administration d'un peptide selon l'une des revendications 1 ou 2 à un animal non-humain et la récupération des anticorps ou des cellules productrices d'anticorps.

8. Méthode de détection de l'AA4RP dans un échantillon biologique comprenant la mise en contact de l'échantillon avec un anticorps selon l'une des revendications 3 à 6 ou avec un anticorps produit grâce à une méthode selon la revendication 7, et la détection de la présence de complexes immuns antigène-anticorps.

9. Méthode selon la revendication 8, **caractérisée en ce que** la présence d'un complexe immun antigène-anticorps est déterminée par EIA, RIA, néphélométrie, turbidimétrie, immunoblot quantitatif, calorimétrie, interférométrie, résonance de surface ou mesure de champ de force.

10. Méthode selon la revendication 8, **caractérisée en ce que** la présence d'un complexe immun antigène-anticorps est déterminée par un test ELISA.

11. Méthode de quantification de l'AA4RP dans un échantillon biologique, comprenant la mise en contact dudit échantillon avec un anticorps selon l'une des revendications 3 à 6 ou avec un anticorps produit par une méthode selon la revendication 7, et la quantification des complexes immuns AA4RP-anticorps.

12. Méthode selon la revendication 11, **caractérisée en ce que** la quantification est réalisée par méthode ELISA.

13. Méthode selon l'une des revendications 8 à 12, **caractérisée en ce que** l'échantillon biologique est un échantillon de sang, de sérum, un fluide interstitiel ou un extrait tissulaire ou cellulaire.

14. Méthode de détection de la présence d'une prédisposition ou d'un risque de développer un désordre lié au métabolisme des lipides chez un sujet, comprenant la quantification *in vitro* ou *ex vivo* des niveaux de l'AA4RP dans un échantillon prélevé sur un sujet, avec un anticorps selon l'une des revendications 3 à 6, ou avec un anticorps produit par une méthode selon la revendication 7.

15. Méthode de suivi d'un traitement destiné à corriger des désordres liés au métabolisme des lipides chez un sujet, comprenant la mesure des quantités de l'AA4RP *in vitro* ou *ex vivo,* dans un échantillon provenant dudit sujet, à l'aide d'un anticorps selon l'une des revendications 3 à 6 ou d'un anticorps produit par une méthode selon la revendication 7.

16. Anticorps selon l'une des revendications 3 à 6 ou produit par une méthode selon la revendication 7, **caractérisé en ce que** l'anticorps est couplé à un fragment hétérologue tel qu'une toxine, un marqueur, un médicament ou tout autre agent thérapeutique.

17. Composition pharmaceutique comprenant un anticorps selon l'une des revendications 3 à 6 et 16 ou produit par une méthode selon la revendication 7 et un excipient ou un véhicule acceptable sur le plan pharmaceutique.

18. Kit comprenant un peptide selon l'une des revendications 1 ou 2 ou un anticorps selon l'une des revendications 3 à 6 et 16 ou produit par une méthode selon la revendication 7.

## Claims

1. Substantially pure synthetic peptide, wherein it consists in the amino acid sequence SEQ ID NO : 2.

2. Peptide according to claim 1, wherein the peptide is soluble or complexed with a carrier molecule such as KLH, serum albumin or a bead.

3. Antibody specific of a peptide according to any one of claims 1 or 2, or fragment or derivative of said antibody having substantially the same antigenic specificity.

4. Antibody according to claim 3, wherein it is produced by immunizing a non-human animal with a peptide according to any one of claims 1 or 2, and recovering antibodies or antibody-producing cells.

5. Antibody according to any one of claims 3 or 4, wherein it is a polyclonal antibody.

6. Antibody according to any one of claims 3 or 4, wherein it is a monoclonal antibody.

7. Method for producing a specific anti-AA4RP antibody, comprising administering a peptide according to any one of claims 1 or 2 to a non-human animal and recovering antibodies or antibody-producing cells.

8. Method for detecting AA4RP in a biological sample comprising contacting the sample with an antibody according to any one of claims 3 to 6 or with an antibody produced by a method according to claim 7, and detecting the presence of antigen-antibody immune complexes.

9. Method according to claim 8, wherein the presence of an antigen-antibody immune complex is determined by EIA, RIA, nephelometry, turbidimetry, quantitative immunoblot, calorimetry, interferometry, surface resonance or force field measurement.

10. Method according to claim 8, wherein the presence of an antigen-antibody immune complex is determined by an ELISA test.

11. Method for quantifying AA4RP in a biological sample, comprising contacting said sample with an antibody according to any one of claims 3 to 6 or with an antibody produced by a method according to claim 7, and quantifying the AAR4P-antibody immune complexes.

12. Method according to claim 11, wherein the quantification is carried out by an ELISA method.

13. Method according to any one of claims 8 to 12, wherein the biological sample is a sample of blood, serum, interstitial fluid or a tissue or cellular extract.

14. Method for detecting the presence of a predisposition or a risk of developing a disorder of lipid metabolism in a subject, comprising quantifying *in vitro* or *ex vivo* the levels of AA4RP in a sample from a subject, with an antibody according to any one of claims 3 to 6, or with an antibody produced by a method according to claim 7.

15. Method for monitoring a treatment for correcting disorders of lipid metabolism in a subject, comprising measuring the quantities of AA4RP *in vitro* or *ex vivo* in a sample from said subject, by means of an antibody according to any one of claims 3 to 6, or of an antibody produced by a method according to claim 7.

16. Antibody according to any one of claims 3 to 6 or produced by a method according to claim 7, wherein the antibody is coupled to a heterologous fragment such as a toxin, a marker, a medicament or any other therapeutic agent.

17. Pharmaceutical composition comprising an antibody according to any one of claims 3 to 6 and 16 or produced by a method according to claim 7 and a pharmaceutically acceptable excipient or vehicle.

18. Kit comprising a peptide according to any one of claims 1 or 2 or an antibody according to any one of claims 3 to 6 and 16 or produced by a method according to claim 7.

## Patentansprüche

1. Im Wesentlichen reines synthetisches Peptid, **dadurch gekennzeichnet, dass** es aus der Aminosäure-Sequenz SEQ ID Nr. 2 besteht.

2. Peptid nach Anspruch 1, **dadurch gekennzeichnet, dass** es löslich ist oder komplex gebunden ist an ein Trägermolekül, wie z.B. KLH, Serumalbumin oder eine kleine Kugel.

3. Spezifischer Antikörper eines Peptids nach einem der Ansprüche 1 oder 2 oder Fragment oder Derivat des Antikörpers, der (das) im Wesentlichen die gleiche Antigen-Spezifität aufweist.

4. Antikörper nach Anspruch 3, **dadurch gekennzeichnet, dass** er hergestellt worden ist durch Immunisieren eines Nicht-Human-Tieres mit einem Peptid nach einem der Ansprüche 1 oder 2 und Abtrennen (Gewinnen) der Antikörper oder der Antikörper bildenden Zellen.

5. Antikörper nach einem der Ansprüche 3 oder 4, **dadurch gekennzeichnet, dass** es sich dabei um einen polyklonalen Antikörper handelt.

6. Antikörper nach einem der Ansprüche 3 oder 4, **dadurch gekennzeichnet, dass** es sich dabei um einen monoklonalen Antikörper handelt.

7. Verfahren zur Herstellung eines anti-AA4RP-spezifischen Antikörpers, das umfasst die Verabreichung eines Peptids nach einem der Ansprüche 1 oder 2 an ein Nicht-Human-Tier und die Gewinnung (Abtrennung) der Antikörper oder der Antikörper bildenden Zellen

8. Verfahren zum Nachweis von AA4RP in einer biologischen Probe, das umfasst das Inkontaktbringen der Probe mit einem Antikörper nach einem der Ansprüche 3 bis 6 oder mit einem Antikörper, der nach einem Verfahren nach Anspruch 7 gebildet worden ist, und den Nachweis der Anwesenheit (des Vorliegens) von Antigen-Antikörper-Immunkomplexen.

9. Verfahren nach Anspruch 8, **dadurch gekennzeichnet, dass** die Anwesenheit (das Vorliegen) eines Antigen-Antikörper-Immunkomplexes bestimmt wird durch EIA, RIA, Nephelometrie, Turbidimetrie, quantitativen Immunoblot, Kalorimetrie, Interferometrie, Oberflächenresonanz oder Feldstärken-Messung.

10. Verfahren nach Anspruch 8, **dadurch gekennzeichnet, dass** die Anwesenheit eines Antigen-Antikörper-Immunkomplexes durch einen ELISA-Test bestimmt wird.

11. Verfahren zur quantitativen Bestimmung von AA4RP in einer biologischen Probe, das umfasst das Inkontaktbringen der genannten Probe mit einem Antikörper nach einem der Ansprüche 3 bis 6 oder mit einem Antikörper, der nach einem Verfahren nach Anspruch 7 hergestellt worden ist, und die quantitative Bestimmung der AA4RP-Antikörper-lmmunkomplexe.

12. Verfahren nach Anspruch 11, **dadurch gekennzeichnet, dass** die quantitative Bestimmung nach dem ELISA-Verfahren durchgeführt wird.

13. Verfahren nach einem der Ansprüche 8 bis 12, **dadurch gekennzeichnet, dass** es sich bei der biologischen Probe um eine Blutprobe, Serumprobe, eine interstitielle Flüssigkeit oder einen Gewebe- oder Zellextrakt handelt.

14. Verfahren zum Nachweis des Vorliegens einer Prädisposition oder eines Risikos zur Entwicklung einer Störung, die mit dem Fettstoffwechsel im Zusammenhang steht, bei einem Patienten, das umfasst die quantitative Bestimmung von AA4RP in vitro oder ex vivo in einer einem Patienten entnommenen Probe mit einem Antikörper nach einem der Ansprüche 3 bis 6 oder mit einem Antikörper, der nach einem Verfahren nach Anspruch 7 hergestellt worden ist.

15. Verfahren, das auf eine Behandlung folgt, die dazu dient, Störungen zu korrigieren, die mit dem Fettstoffwechsel eines Patienten im Zusammenhang stehen, wobei das Verfahren umfasst die Messung der AA4RP-Mengen in vitro oder ex vivo in einer aus dem Patienten stammenden Probe mit Hilfe eines Antikörpers nach einem der Ansprüche 3 bis 6 oder eines Antikörpers, der nach einem Verfahren nach Anspruch 7 hergestellt worden ist.

16. Antikörper nach einem der Ansprüche 3 bis 6 oder Produkt, das nach einem Verfahren nach Anspruch 7 hergestellt worden ist, **dadurch gekennzeichnet, dass** der Antikörper an ein heterologes Fragment, beispielsweise ein Toxin, einen Marker, ein Arzneimittel oder irgendein anderes therapeutisches Agens, gekuppelt ist.

17. Pharmazeutische Zusammensetzung, die einen Antikörper nach einem der Ansprüche 3 bis 6 und 16 oder ein nach einem Verfahren nach Anspruch 7 hergestelltes Produkt und einen pharmazeutisch akzeptablen Exzipienten oder Träger (Vehiculum) umfasst.

18. Kit, der ein Peptid nach einem der Ansprüche 1 oder 2 oder einen Antikörper nach einem der Ansprüche 3 bis 6 und 16 oder ein nach einem Verfahren nach Anspruch 7 hergestelltes Produkt umfasst.
